# EUROPEAN PATENT SPECIFICATION

(11) **EP 1 414 433 B1**
(45) Date of publication and mention of the grant of the patent: **12.04.2006**
(21) Application number: 02748487.2
(22) Date of filing: 17.07.2002
(51) Int. Cl.: A61K 31/215, A61K 9/48, A61K 31/00, A61P 3/06

(54) **IMPROVED PHARMACEUTICAL COMPOSITION CONTAINING A PPAR ALPHA AGENT AND A PROCESS FOR PREPARING IT**
VERBESSERTE PHARMAZEUTISCHE ZUSAMMENSETZUNG MIT EINEM PPAR-ALPHA-MITTEL UND VERFAHREN ZU IHRER HERSTELLUNG
COMPOSITION PHARMACEUTIQUE AMELIOREE CONTENANT UN AGENT PPAR ALPHA ET SON PROCEDE DE PREPARATION

(30) Priority: 07.08.2001 WO PCT/BE01/00133; 05.04.2002 WO PCT/BE02/00051
(43) Date of publication of application: 06.05.2004
(73) Proprietor: Laboratoires SMB SA, 1080 Bruxelles (BE)
(72) Inventor: VANDERBIST, Francis, B-1650 Beersel (BE); BAUDIER, Philippe, B-1180 Uccle (BE); SERENO, Antonio, B-1820 Melsbroek (BE)
(74) Representative: Powis de Tenbossche, Roland
(86) International application number: PCT/BE2002/000123
(87) International publication number: WO 2003/013500

(56) References cited:
- WO-A-00/59475
- WO-A-01/21154
- WO-A-99/12528

## Description

### ABSTRACT

An oral semi-solid composition containing at least one peroxisome proliferator activated alpha agent (PPARα) at a therapeutical dose, a polyglycolized glyceride and an hydrophilic disintegrating agent.

### BACKGROUND OF THE INVENTION

Fibrates are old hypolipidemic drugs with pleitropic effects on lipid metabolism. Their intimate molecular mechanisms of action have been mysterious for a long time. Recently, it has been shown that the pharmacological effect of fibrates depends on their binding to "Peroxisome Proliferator Activated Receptor alpha" (PPAR alpha). The binding of fibrates to PPAR induces the activation of the inhibition of multiple genes involved in lipid metabolism through the binding of the activated PPAR alpha to "Peroxisome Proliferator Response Element" (PPRE) located in the gene promoters. Furthermore, it was recently demonstrated that fibrates are potent antiinflammatory molecules through an indirect modulation of the nuclear-factor-Kappa B activity.

Fenofibrate or P-(4-chlorobenzoyl)-phenoxy isobutyrate isopropyl ester is useful for the treatment of adult patients with very high elevations of serum triglyceride levels and/or cholesterol levels. Initially, the usual daily dosage was 300 mg which was administered in two or three doses. A few years later, a suprabioavailable composition of fenofibrate was developed and marketed by Laboratoires Fournier, France (EP 0030532). 200 mg of fenofibrate from this composition was bioequivalent to 300 mg of the initial composition. Fenofibrate is absorbed as fenofibric acid which is responsible for the pharmacological activity. Fenofibric acid resulting from the hydrolysis of fenofibrate is extensively bound to plasma albumin. The plasma half-life is about 20 hours. Fenofibric acid is excreted predominantly in the urine, mainly as the glucuronide conjugate, but also as a reduced form of fenofibric acid and its glucuronides.

Fenofibrate is presently available in a pharmaceutical dosage form consisting of hard gelatin capsules containing fenofibrate, lactose starch and magnesium stearate. After oral administration, during a meal, about 60% of the dose of this conventional form is effectively absorbed and found in the blood as fenofibric acid, the main metabolite responsible for pharmacological activity.

The first attempt to improve the bioavailability of fenofibrate was performed by Ben-Armor et al, by solubilizing the fenofibrate in dimethyl isosorbide, a nonaqueous solvent with a miscible wetting agent (Labrafil M1944CS) with HLB of between 3-4. In order to use the product in capsules, colloidal silicon oxide was added to increase the viscosity. The liquid so obtained was placed in hard gelatin capsules which, to be leak proof, were sealed. In vivo studies with this formulation indicate that there was no statistically significant difference in bioavailability between this liquid formulation and the conventional form when the product was given with food.

European Patent Application 0330532 discloses a fenofibrate composition wherein the fenofibrate powder is co-micronized with a solid wetting agent. Sodium lauryl sulfate is described as the solid wetting agent of choice. The co-micronized powder so obtained is mixed with capsule filling excipient such as lactose, starch, polyvinyl pyrollidone and magnesium stearate. A formulation of this composition is actually available on the French market under the trade name Lypanthyl® 200, Fournier, France. A study comparing this formulation (Lypanthyl® 200) to the conventional form was undertaken and a statistically significant increase in bioavailability was indicated for the former. In particular, it was found that 67 mg of the new form gives the same amount absorbed as does 100 mg of the conventional form.

Unfortunately, co-micronization of the active drug fenofibrate with the wetting agent sodium lauryl sulfate, although necessary, is a time consuming and costly operation. Further, an inherent drawback of micronization is that the material obtained must comply with very stringent particle size specifications. Indeed, the solubility and thus bioavailability depend on the particle size, whereby the presence of only some larger fenofibrate particles in a dosage form containing 160mg could modify greatly the solubility and bioavailability.

The Canadian patent 2,214,895 describes an improved formulation of fenofibrate obtained by making a solid dispersion of fenofibrate and a disintegrating agent.

Moreover, the filling of hard gelatin capsules with a micronized powder is a difficult operation, particularly if weight variation homogeneity is considered.

Hence, a need exists for a fenofibrate formulation that avoids the use of co-micronization, while providing a bioavailability comparable to that afforded by the conventional fenofibrate formulation which uses co-micronization.

Consequently, there was a need to dispose of a fenofibrate composition easy to manufacture and providing a high bioavailability after oral intake. Such a composition is disclosed in US patent 5,545,628 based on a semi-solid formulation containing fenofibrate and an excipient containing one or more polyglycolized glycerides.

But, more recently, after that, the US patent 6,277,405 describes a composition of fenofibrate allowing to still improve the bioavailability of fenofibrate after oral intake. This patent describes the use of:
a) an inert hydrosoluble carrier covered with at least one layer containing fenofibrate active ingredient in a micronized form having a size smaller than 20 microns (µm), a hydrophilic polymer and, optionally, a surfactant, said hydrophilic polymer making up at least 20% by weight of an (a) and :
b) optionally one or several outer phase(s) or layer(s).

The composition described in the US patent 6,277,405 allows indeed to improve the bioavailability of fenofibrate in comparison with the commercial forms available on the market for several years (TRICOR® 200, LIPIDIL® 200, LIPANTHYL® 200, Fournier, France) and corresponding to the older US patent EP 0330532.

Pharmacokinetic studies have demonstrated that 200 mg of the commercial formulation are equivalent to 160 mg of the composition corresponding to the patent 6,277,405 (i.e. a 20% increase of bioavailability), which is already commercialized in several countries (LIPIDIL® 160, LIPANTHYL® 160).

Nevertheless, the process and the composition described in the patent 6,277,405 are complex, difficult and long to manufacture, and expensive. There was therefore still a need for a fenofibrate oral composition allowing to reach a similar bioavailability as that of US patent 6,277,405 but with a simpler and cheaper manufacturing process. The formulation described in US patent 5,545,628 did not offer a sufficient bioavailability. Indeed, this kind of composition gives a bioavailability similar to that of Tricor® 200 mg but 20% inferior to the LIPIDIL® 160 mg.

### SUMMARY OF THE INVENTION

It is an object of the present invention to provide a PPARα agent containing composition (preferably a fenofibrate containing composition) with a very high bioavailability, which can be prepared without requiring micronisation and/or the presence of an hydrophilic polymer and/or the necessity of an outer phase layer. It is also an object of the invention to describe an easy, reliable and cheap process for manufacturing the new composition.

### DETAILED DESCRIPTION OF THE PREFERRED EMBODIMENTS

Oral semi-solid pharmaceutical compositions present various advantages:

The advantages of the semi-solid formulations are multiple for a PPAR derivative: protection of the active ingredient from air and humidity, possibility of increasing the dissolution rate of the molecule and hence of bioavailability, diminution of the risk of contamination of the operator, diminution of the risk of cross contamination, no possibility of demixing under the effect of vibrational mixing during manufacturing process, facility of the production process. The choice of the nature of the formulation of course influences the stability of the pharmaceutical form and the bioavailability of the drug contained in it. Generally, a maximum bioavailability is achieved by preparing and keeping the drug in the amorphous/solubilized state in a solid dispersion or in a lipid-based formulation. For these systems, the barrier we are avoiding is the compound « washing-out » of solution to a large extent into a insoluble crystalline form during the dissolution/release step in vivo.

The use of polyglycolized glycerides in this kind of semi-solid compositions has already been described in US patent 5,545,628.

It has now been observed that by using simultaneously a polyglycolized glyceride and a hydrophilic disintegrating agent in a semi-solid or liquid fenofibrate composition, it was possible reach the bioavailability and the mean plasma concentration of the commercial Lipidil® formulation, the latter formulation requiring a co-micronization step as taught in EP 0330532.

The present invention provides a pharmaceutical formulation for treating hyperlipidemia and/or hypercholesterolemia in a mammal, which contains an effective amount of a PPARα agent or a mixture of PPARα agents, advantageously fenofibrate, an excipient which contains one or more polyglycolyzed glycerides, the polyglycolyzed glycerides preferably having an HLB value of at least about 10, and one hydrophilic disintegrating agent.

PPAR agents are agents having a "peroxisome proliferation activated receptor activating effect", i.e. agents activating the binding of PPAR to peroxisome proliferator response element (PPRE), as well as agents activating the PPAR for its binding to PPRE.The agent as such, or a metabolite thereof , or a compound generated by the organism due to the presence of the agent is bound to the PPAR, whereby inducing the binding of PPAR to PPRE.

PPAR agents (alpha, delta and/or gamma) are agents for which the data determined by measuring the receptor-activating effect of a certain compound are statistically judged as being significantly different from the control data determined in the absence of the compound. Many documents disclose PPAR agents. For example, reference can be made to US 6,365,586;US 6,331,627 ; US 6,214,820, etc. disclosing or referring to such agents.

In the composition of the invention, the PPAR agent is advantageously an agent with a low water solubility, such as a water solubility similar or smaller to the water solubility of fenofibrate (for example a water solubility expressed in g/l corresponding to less than 5 (preferably less than 2) times the solubility of fenofibrate in water (pH 7, temperature 20°C).

The PPAR agent is more precisely a PPARα agent, such as a compound of the fibrate family, such as fenofibrate, ciprofibrate, Clofibrate, Gemfibrozil, Bezafibrate or combinations thereof, fenofibrate and combinations containing fencfibrate being most preferred.

The present invention is also particularly advantageous for the production of oral solid dosage forms which can be prepared by melting the excipients in which the fenofibrate is at least partially soluble, whereby particle size specifications are not required.

Advantageously, the present invention also relates to the addition of a suspension stabilizer to the molten solution of PPARα (such as fenofibrate)-polyglycolyzed glycerides. The suspension stabilizer avoids the formation of PPARα crystals, such as fenofibrate crystals, during the cooling of the filled hard gelatin capsules. Suitable suspension stabilizers which may be used are, for example, cellulose derivatives, such as hydroxypropylcellulose, hydroxypropylmethyl cellulose, methyl cellulose, and hydroxyethylcellulose, povidone, poloxamers, .alpha., .OMEGA.-hydroxy-poly(oxyethylene) poly(oxypropylene)-poly(oxyethylene)bloc polymers. Other suspension stabilizers equivalent to these stabiliers may, of course, also be used.

The present invention is also particularly advantageous for the production of a pharmaceutical composition in that the hot, homogeneous PPARα (such as fenofibrate) solution is filled in hard gelatin capsules. This filling process permits to ensure a very precise PPARα (such as fenofibrate) amounts in each capsule.

The present invention is particularly advantageous as well for the production of the present pharmaceutical composition in that the process for manufacturing the composition requires very few steps such as melting, mixing and filling. This renders the present manufacturing process extremely cost effective when compared to one using co-micronization of powders.

Polyglycolyzed glycerides which may be used in the present invention are generally mixtures of known monoesters, diesters and triesters of glycerols and known monoesters and diesters of polyethylene glycols with a mean relative molecular mass between about 200 and 6000. They may be obtained by partial transesterification of triglycerides with polyethylene glycol or by esterification of glycerol and polyethylene glycol with fatty acids using known reactions. Preferably, the fatty acid component contains 8-22 carbon atoms, particularly 10-18 carbon atoms. Examples of natural vegetable oils which may be used include palm kernel oil and palm oil. However, these are only examples. The polyol suitably has a molecular weight in the range of about 200-6000 and preferably contains polyethylene glycol, although other polyols may be employed, such as polyglycerols or sorbitol. They are available on the market under the trade name Gelucire® (Gattefossé, France).

As noted above, the HLB of the polyglycolized glycerides is preferably at least about 10, and more preferably between about 12 and 15. The melting point of the polyglycolized glycerides may be between about 18.degree. C. and 60.degree. C. However, it is especially desirable to use polyglycolized glycerides having a melting point above 30.degree. C., and preferably above 35.degree. C., since there is no need for sealing the capsule, to assure the leak proofness thereof, when such excipients are used.

Further, two or more polyglycolized glycerides may be mixed in order to adjust both the HLB value and the melting point to a desired value. The HLB value and melting point of the composition may further be adjusted with the addition of components such as polyethylene glycols, polyoxyethylene glycols fatty acid esters, and fatty acid alcohols. In view of the present specification, it is well within the skill of the artisan to mix the polyglycolized glycerides to obtain desired HLB values and melting points.

Although the present invention is not bind by any particular theories, one plausible mechanism of operation for the present invention is that upon cooling, the melted mixture of hot PPARα (such as fenofibrate)-polyglycolized glycerides maintains the PPARα (such as fenofibrate) in liquid form. When absorbed in the gastrointestinal tract of a patient, the gastrointestinal fluids are able to dissolve the PPARα (such as fenofibrate) due to the HLB value of the excipient mixture, whereby PPARα (such as fenofibrate) is readily absorbed.

The release of the drug from this kind of semi-solid lipophilic matrix is operated by a phenomenon of erosion-diffusion of the form when it is in contact with the gastro-intestinal fluids. As most excipients have relatively lipophilic properties, the release of the drug from the composition is relatively slow.

It was consequently a goal of the present invention to increase the either the rate of release or the nature of the particles release (size) of PPARα (such as fenofibrate) from this kind of composition.

Surprisingly enough, this increase of release has been obtained by adding in the formulation very hydrophilic excipients (disintegrating agents) not soluble in the fatty mass. Such hydrophilic excipients may be the following ones but are not restricted to them: sodium starch glycolate, sodium croscarmellose, crospovidone, pregelatinized starch, maize starch, Aerosil® (colloidal silicone dioxide). This increase of the rate or quality of release of the drug with formulations containing a disintegrant in a semi-solid formulation is quite surprising since disintegrants are usually used in tablets formulations wherein the forces of compression are quite high.
These kinds of hydrophilic substances while not soluble are able to be dispersed homogeneously in the fatty mass and remain homogeneously dispersed after capsule filling and cooing.

The invention relates also to the use of a composition according to the invention for the manufacture of a medicament for treating or preventing hyperlipidemia or hypercholesterolemia in a patient, in which at least an effective amount of a peroxisome proliferator activated receptor agent is orally administered to the patient simultaneously with at least one polyglycolized glyceride and one hydrophilic disintegrating agent. In this method, the PPAR agent, the polyglycolized glyceride and the disintegrating agent are preferably of the type disclosed for the composition of the invention.

The invention further relates to a process for the preparation of an oral semi-solid or liquid composition containing at least an effective amount of PPAR agent, at least one polyglycolised glyceride and one hydrophilic disintegrating agent, in which the PPAR agent in powder form and hydrophilic disintegrating agent are mixed to a molten mixture containing at least one polyglycolised glyceride.
Advantageously, the PPAR agent and the hydrophilic disintegrating agent are mixed successively with the molten mixture.
Preferably, the PPAR agent is first mixed with the molten mixture, and before adding the hydrophilic disintegrating agent, the homogeneous dispersion of the PPAR powder in the molten mixture is controlled

The weight ratio polyglycolized glyceride(s)/hydrophilic disintegrating agent in the composition of the invention or in the method of the invention or in the process of the invention is for example comprised between 100 and 0.1, advantageously between 50 and 2, preferably between 40 and 4, for example between 8 and 25.

The weight ratio PPAR agent/hydrophilic disintegrating agent in the composition of the invention or in the method of the invention or in the process of the invention is for example comprised between 100 and 0.1, advantageously between 50 and 2, preferably between 40 and 4, for example between 6 and 25.

The weight ratio PPAR agent/polyglycolized glyceride(s) in the composition of the invention and in the method of the invention or in the process of the invention is for example comprised between 10 and 0.1, advantageously between 5 and 0.5, preferably greater than 1, such as between 1.1 and 2, for example 1.2, 1.5, etc.

### BRIEF DESCIPTION OF THE DRAWINGS

Figure 1 is a graph showing the in vitro comparative dissolution profiles of 2 semi-solid formulations of fenofibrate with and without disintegrant
Figure 2 is a graph showing the in vitro comparative dissolution profiles of 2 semi-solid fenofibrate compositions containing different disintegrating agents
Figure 3 is a graph showing the pharmacokinetic results of comparative study in 18 volunteers for fenofibrate (log-transformed data).

### EXAMPLES

An example of preferred manufacturing process is given hereinbelow:

The manufacturing process allowing to obtain the composition described in the present invention is, for example, as given hereinbelow. In this example, fenofibrate Is used as example of PPARα agent.

Melt the Gelucire® 44/14 and PEG in an adequately equipped mixer for liquid (TRIAGI, LLEAL Barcelone, Spain, or FRYMA, Basel, Switzerland) at 75°C. Add the fenofibrate powder to the molten mix. Continue the mixing while mounting the temperature of the mix to 75°C. Once fenofibrate is homogeneously dissolved in the mass, add the hydropropylcellulose at 75°C and under mixing. Finally add the disintegrating agent at 75°C and under mixing. The disintegrating agent is not dissolved In the fatty excipients but is homogeneously dispersed in the mass. Start the filling of hard gelatine or hypromellose capsules (at 70°C). Allow the capsules to cool until 30°C. At this temperature, the product becomes solid. Package adequately the capsules.

Different examples of compositions 1 to 5 are given hereinbelow:

| | |
|---|---|
| Fenofibrate | 200 |
| Saturated polyglycolized glycerides (Gelucire 44/14) | 250 |
| Polyethyleneglycol (PEG) 8000 | 55 |
| Hydropropylcellulose (HPC) | 95 |
| Sodium starch glycolate (Explotab) | 20 |
| | **620 mg (composition 1)** |

| | |
|---|---|
| Fenofibrate | 267 |
| Saturated polyglycolized glycerides (Golucire 44/14) | 290 |
| Polyethyleneglycol (PEG) 8000 | 55 |
| Hydropropylcellulose (HPC) | 95 |
| Polyplasdone XL (PLPXL) | 20 |
| | **727 mg (composition 2)** |

| | |
|---|---|
| Fenofibrate | 200 |
| Saturated polyglycolized glycerides (Gelucire 44/14) | 290 |
| Polyethyleneglycol (PEG) 10000 | 30 |
| Polyethyleneglycol (PEG) 20000 | 30 |
| Hydropropylcellulose (HPC) | 90 |
| Sodium starch glycolate (Explotab) | 20 |
| | **660 mg (composition 3)** |

| | |
|---|---|
| Fenofibrate | 160 |
| Saturated polyglycolized glycerides (Gelucire 44/14) | 240 |
| Polyethyleneglycol (PEG) 20000 | 48 |
| Hydropropylcellulose (HPC) | 95 |
| Sodium starch glycolate (Explotab) | 20 |
| | **563 mg (composition 4 or Fenogal® Lidose®)** |

| | |
|---|---|
| Fenofibrate | 200 |
| Saturated polyglycolized glycerides (Gelucire 44/14) | 300 |
| Polyethyleneglycol (PEG) 20000 | 55 |
| Hydropropylcellulose (HPC) | 95 |
| Croscarmellose (AC-Dl-SOL) | 20 |
| | **670 mg (composition 5)** |

Compositions similar to compositions 1 to 5 have been prepared by replacing the fenofibrate powder by other PPAR agents, namely clofibrate, bezafibrate, ciprofibrate, gemfibrozil, mixtures of PPAR agents, such as mixture of bezafibrate (10-90%) + fenofibrate (90-10%). The following composition 6 is a specific example of composition containing clofibrate.

| | |
|---|---|
| clofibrate | 300 |
| Saturated polyglycolized glycerides (Gelucire 44/14) | 340 |
| Polyethyleneglycol (PEG) 20000 | 80 |
| Hydropropylcellulose (HPC) | 125 |
| Sodium starch glycolate (Explotab) | 20 |
| | **865 mg (composition 6)** |

### Effect of the presence of an disintegrating agent

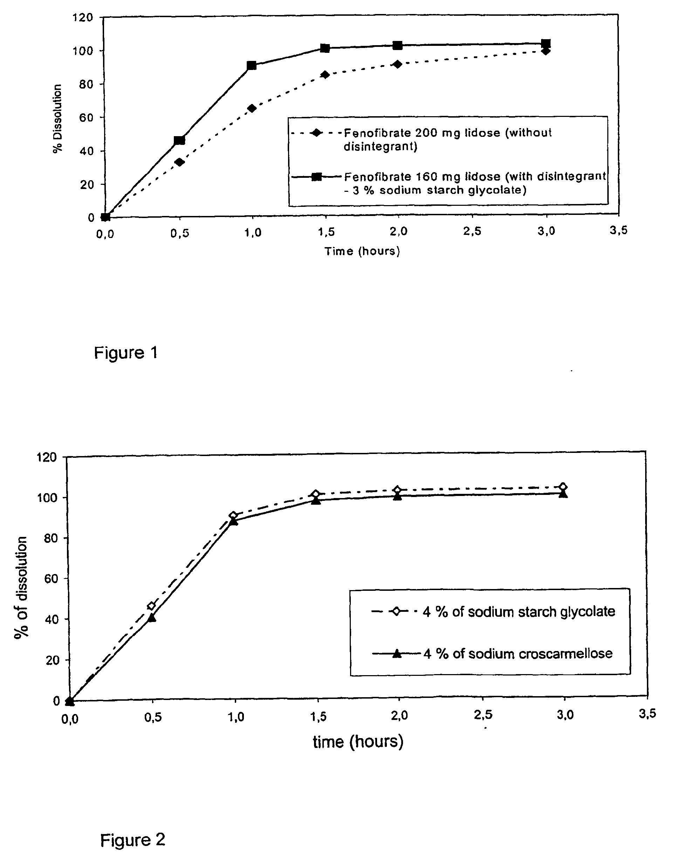

An in vitro dissolution test has been performed to assess if the presence of a disintegrating agent in the formulation allowed to increase the release and / or the dissolution of fenofibrate. As seen in figure 1, the presence of 3 % of sodium strarch glycolate clearly increases the dissolution rate of fenofibrate.

In said figure 1, the in vitro comparative dissolution profiles of 2 semi-solid formulations of fenofibrate with and without disintegrant are shown.

The dissolution method used for said comparative study is described herebelow.
- Volume of dissolution: 900 ml
- Test temperature: 37.0 ± 0.5°C.
- Paddle rotation speed: 100 rpm.
- Test duration: 180 minutes.
- Detection : UV at 288 nm.
- Blank : dissolution medium.
- n = 6 vessels / test

The dissolution medium was 900 ml of an acidic (pH 1.2) dissolution medium containing Polysorbate 80 (2 %) as surfactant and pepsin (3.2 g/l) as enzyme.

### Effect of the type of disintegrating agent

It was also interesting to assess if different disintegrating agents substances were able to increase the release and/or dissolution of fenofibrate in vitro in comparison in the same way. Therefore, the dissolution profile of a semi-solid formulation of fenofibrate containing 4% of sodium starch glycolate was compared with a semi-solid formulation of fenofibrate containing 4% of sodium croscarmellose.

Figure 2 shows the in vitro comparative dissolution profiles of 2 semi-solid fenofibrate compositions containing different disintegrating agents (dissolution method - see hereinabove).
It appears from said figure that sodium croscarmellose and sodium starch glycolate give similar increase of dissolution rate.

But an increase of the dissolution rates does not mean that the composition corresponding to the present invention allows to increase the bioavailability of fenofibrate in humans after an oral administration. Therefore, pharmacokinetic studies have been performed:

### In vivo test

The bioavailability of FENOGAL® LIDOSE® 160 mg capsule (Laboratoires SMB SA) has been assessed and compared to the bioavailability of the reference (LIPIDIL-TER® 160 mg tablet, Fournier) on 18 healthy subjects. LIPIDIL-TER® 160 mg tablet is a suprabioavailable formulation of fenofibrate wherein the higher bioavailability of the drug is obtained by comicronizing the fenofibrate together with a surfactant. LIPIDIL-TER® 160 mg is already on the market in several countries.
This study (SMB-FENO-SD012) was a single dose, two treatments, two periods, two sequences, randomised, cross-over and with at least 8 days wash-out between the two periods.
The subjects were healthy Caucasian volunteers of both sexes (non-pregnant, non-breast-feeding), aged 18 to 50 years, non smokers or smoking less than 10 cigarettes per day.
The drugs (one tablet or one capsule containing 160 mg of fenofibrate in one intake during the two periods) were taken with food (a standardized breakfast).
Blood samples were collected according to the following sampling schedule: pre-dose and 1 h, 2h, 3h, 4h, 5h, 6h, 7h, 9h, 12h, 24h, 36h, 48h, 60h and 72 hours post-dose.
The plasma concentrations of fenofibrate were quantified using a fully validated LC/MS/MS method.

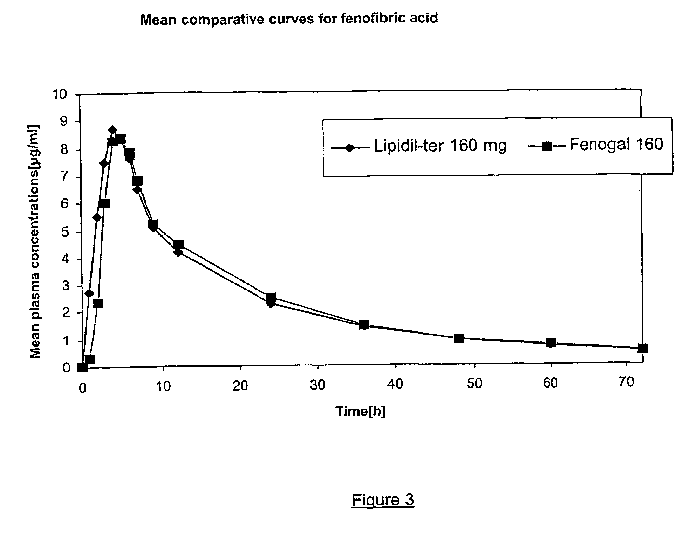

The figure 3 describes the mean pharmacokinetic profile of fenofibrate for the two formulations (n=18 subjects).

The continuous variables (AUC_{T}, AUC_{∞}, Cₘₐₓ, t_{1/2} and MRT) were evaluated according to an univariate ANOVA based on log-transformed data. For Tₘₐₓ, the non parametric Kruskal-Wallis test was used. Bioequivalence was evaluated using the Shuirman two one-sided t-test (90% Cl). The Kinetica Program (Innaphase®) has been applied for this calculations.

The table hereinbelow gives the value of the main pharmacokinetics results and statistical analysis obtained for each formulation of fenofibrate.

**Table 1 : Pharmacokinetic results and statistical analysis of comparative study in 18 volunteers for fenofibrate (log-transformed data)**

| Results | | | Bioequivalence tests |
|---|---|---|---|
| Parameter | LIPIDIL-TER® 160 mg | FENOGAL® LIDOSE® 160 mg | Shuirman 90% Cl Range (acceptance range : 80-125) |
| AUC_{∞} | 173.29 | 172.43 (µg.h/ml) | 94-105 |
| ± SD | (µg.h/ml) | ± 85.78 | |
| | ± 90.82 | | |
| AUC_{T} | 161.21 | 160.42 (µg.h/ml) | 94-105 |
| ± SD | (µg.h/ml) | ± 70.52 | |
| | ± 74.30 | | |
| Cₘₐₓ | 9.54 (µg /ml) | 9.12 (µg /ml) | 88-103 |
| ±SD | ±1.97 | ± 2.10 | |
| Tₘₐₓ | 4.17 (h) | 4.50 (h) | / |
| ±SD | ± 1.38 | ± 0.98 | |
| t_{1/2} | 15.57 (h) | 15.58 (h) | 95-106 |
| ± SD | ± 4.56 | ± 4.37 | |
| MRT | 23.05 (h) | 23.91 (h) | 99-110 |
| ± SD | ± 7.14 | ± 6.92 | |

This study demonstrated that LIPIDIL-TER® 160 mg and FENOGAL® LIDOSE® 160 mg are bioequivalent after a single oral dose administration of each product in fed conditions. Indeed, the pharmacokinetics parameters AUC (AUC_{∞} and AUC_{T} ), Cₘₐₓ , Tₘₐₓ , t_{1/2} and MRT were within the predetermined confidence interval. Consequently, the present invention allows to obtain a similar suprabioavailable product as LIPIDIL-TER® 160 mg but with a very simplier and cheaper manufacturing process.

## Claims

1. An oral semi-solid or liquid composition for treating hyperlipidemia of hypercholesterolemia in humans, which comprises : (a) at least an effective amount of a peroxisome proliferator activated receptor alpha agent (PPARα) of the fibrate family, preferably a compound selected from the group consisting of fenofibrate, ciprofibrate, clofibrate, gemfibrozil, bezafibrate and combinations thereof; (b) one polyglycolized glyceride, and (c) one hydrophilic disintegrating agent homogeneously dispersed in the composition, wherein the disintegrating agent is sodium croscarmellose, crospovidone, starch, colloidal silicone dioxide, or sodium starch glycolate.

2. The composition of claim 1, wherein the PPARα agent is fenofibrate.

3. The composition of claim 1, wherein the PPARα is clofibrate.

4. The composition of claim 1 wherein the PPARα is bezafibrate or ciprofibrate.

5. The composition of claim 1, wherein the disintegrating agent is sodium starch glycolate.

6. The composition of claim 1 further containing a polyethylene glycol or a mix of polyethylene glycol with different molecular mass.

7. The composition of claim 1, wherein a suspension stabilizer is added in the composition.

8. The composition of claim 7, wherein the suspension stabilizer is a cellulose derivative.

9. The composition of claim 8, wherein the suspension stabilizer is hydropropylcellulose.

10. The composition of claim 2. wherein said fenofibrate is present in amount of 10% to 80% per weight based on the total weight of the formulation.

11. The composition of claim 2. wherein the amount of fenofibrate per dose is between 30 and 400 mg.

12. The composition of claim 1, wherein the composition is filled in hard gelatine capsules, hypromellose capsules or in other pharmaceutically acceptable capsules.

13. The composition of claim 2, which is with the proviso that the fenofibrate is not co-micronized.

14. The composition of claim 1, in which the weight ratio PPAR agent/hydrophilic disintegrating agent is comprised between 100 and 0.1, advantageously between 50 and 2, preferably between 40 and 4, more preferably between 6 and 25, while the weight ratio PPAR agent/polyglycolized glyceride(s) is comprised between 10 and 0.1, advantageously between 5 and 0.5, preferably greater than 1, more preferably between 1.1 and 2.

15. A process for the preparation of an oral semi-solid or liquid composition containing at least an effective amount of PPAR agent, at least one polyglycolised glyceride and one hydrophilic disintegrating agent, in which the PPAR agent in powder form and hydrophilic disintegrating agent are mixed to a molten mixture containing at least one polyglycolised glyceride.

16. The process of claim 15, in which the PPAR agent and the hydrophilic disintegrating agent are mixed successively with the molten mixture.

17. The process of claim 16, in which the PPAR agent is first mixed with the molten mixture, and in which before adding the hydrophilic disintegrating agent, the homogeneous dispersion of the PPAR powder in the molten mixture is controlled.

## Patentansprüche

1. Halbfeste oder flüssige Zusammensetzung zum Behandeln einer Hyperlipidämie oder Hypercholesterinämie bei Menschen, die folgendes umfaßt: (a) Mindestens eine wirksame Menge eines Peroxisomenproliferator-aktivierten Rezeptor alpha-Wirkstoffs *(peroxisome proliferator-activated receptor α*, PPAR*α*) der Fibrat-Familie, vorzugsweise einer Verbindung, die ausgewählt ist aus der Gruppe, bestehend aus Fenofibrat, Ciprofibrat, Clofibrat, Gemfibrozil, Bezafibrat und Kombinationen davon, (b) ein polyglykosiliertes Glyzerid und (c) ein hydrophiles Sprengmittel, das in der Zusammensetzung homogen dispergiert ist, wobei das Sprengmittel Natriumcroscarmellose, Crospovidon, Stärke, kolloidales Silikondioxid oder Natriumstärkeglykolat ist.

2. Zusammensetzung nach Anspruch 1, wobei der PPARα-Wirkstoff Fenofibrat ist.

3. Zusammensetzung nach Anspruch 1, wobei der PPARα Clofibrat ist.

4. Zusammensetzung nach Anspruch 1, wobei der PPARα Bezafibrat oder Ciprofibrat ist.

5. Zusammensetzung nach Anspruch 1, wobei das Sprengmittel Natriumstärkeglykolat ist.

6. Zusammensetzung nach Anspruch 1, die weiterhin ein Polyethylenglykol oder eine Mischung aus Polyethylenglykol mit verschiedenen Molekulargewichten enthält.

7. Zusammensetzung nach Anspruch 1, wobei der Zusammensetzung ein Suspensionsstabilisator zugesetzt wird.

8. Zusammensetzung nach Anspruch 7, wobei der Suspensionsstabilisator ein Zellulosederivat ist.

9. Zusammensetzung nach Anspruch 8, wobei der Suspensionsstabilisator Hydropropylzellulose ist.

10. Zusammensetzung nach Anspruch 2, wobei das genannte Fenofibrat in einer Menge von 10 Gew.-% bis 80 Gew.-%, bezogen auf das Gesamtgewicht der Formulierung, vorhanden ist.

11. Zusammensetzung nach Anspruch 2, wobei die Menge an Fenofibrat pro Dosis zwischen 30 und 400 mg beträgt.

12. Zusammensetzung nach Anspruch 1, wobei die Zusammensetzung in Hartgelatinekapseln, Hypromellosekapseln oder in andere pharmazeutisch zulässige Kapseln gefüllt wird.

13. Zusammensetzung nach Anspruch 2, welche die Bedingung erfüllt, daß Fenofibrat nicht co-micronisiert ist.

14. Zusammensetzung nach Anspruch 1, wobei das Gewichtsverhältnis von PPAR-Wirkstoff/hydrophiles Sprengmittel zwischen 100 und 0,1, vorteilhafterweise zwischen 50 und 2, vorzugsweise zwischen 40 und 4, stärker bevorzugt zwischen 6 und 25 beträgt, während das Gewichtsverhältnis von PPAR-Wirkstoff/polyglykolysiertem Glyzerid bzw. PPAR-Wirkstoff/polyglykolisierten Glyzeriden zwischen 10 und 0,1, vorteilhafterweise zwischen 5 und 0,5, vorzugsweise größer als 1, stärker bevorzugt zwischen 1,1 und 2 beträgt.

15. Verfahren zur Herstellung einer oralen halbfesten oder flüssigen Zusammensetzung, die mindestens eine wirksame Menge eines PPAR-Wirkstoff, mindestens ein polyglykolysiertes Glyzerid und ein hydrophiles Sprengmittel umfaßt, wobei der PPAR-Wirkstoff in Form eines Pulvers und das hydrophile Sprengmittel zu einer geschmolzenen Mischung, die mindestens ein polyglykosyliertes Glyzerid enthält, gemischt werden.

16. Verfahren nach Anspruch 15, bei dem der PPAR-Wirkstoff und das hydrophile Sprengmittel nacheinander mit der geschmolzenen Mischung gemischt werden.

17. Verfahren nach Anspruch 16, bei dem der PPAR-Wirkstoff zuerst mit der geschmolzenen Mischung gemischt wird und bei dem vor dem Zugeben des hydrophilen Sprengmittels die homogene Dispersion des PPAR-Pulvers in der geschmolzenen Mischung überprüft wird.

## Revendications

1. Composition orale semi-solide ou liquide pour le traitement de l'hyperlipidémie ou de l'hypercholestérolémie chez l'homme, qui comprend : (a) au moins une quantité efficace d'un agent du récepteur alpha activé par des proliférateurs de peroxysome (PPARα) de la famille des fibrates, de préférence un composé choisi parmi le groupe constitué du fénofibrate, du ciprofibrate, du clofibrate, du gemfibrozil, du bézafibrate et des combinaisons de ceux-ci, (b) un glycéride polyglycolysé, et (c) un agent désintégrant hydrophile dispersé dans la composition, où l'agent désintégrant est la croscarmellose de sodium, la crospovidone, l'amidon, le dioxyde de silicium colloïdal ou le glycolate d'amidon de sodium.

2. Composition selon la revendication 1, dans laquelle l'agent PPARα est le fénofibrate.

3. Composition selon la revendication 1, dans laquelle le PPARα est le clofibrate.

4. Composition selon la revendication 1, dans laquelle le PPARα est le bézafibrate ou le ciprofibrate.

5. Composition selon la revendication 1, dans laquelle l'agent désintégrant est le glycolate d'amidon de sodium.

6. Composition selon la revendication 1, contenant en plus un polyéthylèneglycol ou un mélange de polyéthylèneglycols avec des masses moléculaires différentes.

7. Composition selon la revendication 1, dans laquelle un stabilisant de suspension est ajouté dans la composition.

8. Composition selon la revendication 7, dans laquelle le stabilisant de suspension est un dérivé de cellulose.

9. Composition selon la revendication 8, dans laquelle le stabilisant de suspension est de l'hydropropylcellulose.

10. Composition selon la revendication 2, dans laquelle ledit fénofibrate est présent dans une quantité de 10% à 80% en poids du poids total de la formulation.

11. Composition selon la revendication 2, dans laquelle la quantité de fénofibrate par dose est entre 30 et 400 mg.

12. Composition selon la revendication 1, dans laquelle la composition est remplie dans des capsules de gélatine dure, des capsules d'hydromellose ou dans d'autres capsules pharmaceutiquement acceptables.

13. Composition selon la revendication 2, qui remplit la condition que le fénofibrate ne soit pas co-micronisé.

14. Composition selon la revendication 1, dans laquelle le rapport en poids de l'agent PPAR/agent désintégrant hydrophile est compris entre 100 et 0,1, avantageusement entre 50 et 2, de préférence entre 40 et 4, davantage préféré entre 6 et 25, pendant que le rapport en poids de l'agent PPAR/glycéride(s) polyglycolysé(s) est compris entre 10 et 0,1, avantageusement entre 5 et 0,5, de préférence plus grand que 1, davantage préféré entre 1,1 et 2.

15. Procédé de préparation d'une composition orale semi-solide ou liquide contenant au moins une quantité efficace de l'agent PPAR, au moins un glycéride polyglycolysé et un agent désintégrant hydrophile, dans lequel l'agent PPAR sous forme de poudre et l'agent désintégrant hydrophile sont mélangés à un mélange fondu contenant au moins un glycéride polyglycolysé.

16. Procédé selon la revendication 15, dans lequel l'agent PPAR et l'agent désintégrant hydrophile sont mélangés successivement avec le mélange fondu.

17. Procédé selon la revendication 16, dans lequel l'agent PPAR est d'abord mélangé avec le mélange fondu, et dans lequel avant addition de l'agent désintégrant hydrophile, la dispersion homogène de la poudre de PPAR dans le mélange fondu est contrôlée.
